# EUROPEAN PATENT SPECIFICATION

(11) **EP 1 294 358 B1**
(45) Date of publication and mention of the grant of the patent: **18.08.2004**
(21) Application number: 01984051.1
(22) Date of filing: 22.06.2001
(51) Int. Cl.: A61K 9/14

(54) **WET MILLING PROCESS**
NASSVERMAHLUNG
PROCEDE DE BROYAGE PAR VOIE HUMIDE

(30) Priority: 28.06.2000 GB 0015856; 22.05.2001 GB 0112496
(43) Date of publication of application: 26.03.2003
(73) Proprietor: SMITHKLINE BEECHAM PLC, Brentford, Middlesex TW8 9GS (GB)
(72) Inventor: HOLLAND, Simon, Joseph, GlaxoSmithKline, Harlow, Essex CM19 5AW (GB); KNIGHT, Wendy, Anne, GlaxoSmithKline, Harlow, Essex CM19 5AW (GB); LEONARD, Graham, Stanley, GlaxoSmithKline, Harlow, Essex CM19 5AW (GB)
(74) Representative: West, Vivien
(86) International application number: PCT/EP2001/007085
(87) International publication number: WO 2002/000196

(56) References cited:
- EP-A- 0 600 528
- WO-A-01/13889
- WO-A-99/30687

## Description

The present invention relates to the field of milling. More specifically, the present invention relates to a novel milling process which may be used to manufacture submicron particles of a drug substance.

One important criterion for a drug substance is to achieve good bioavailability, this being the degree to which a drug substance is absorbed into the bloodstream after administration, which is usually by the oral route. A variety of factors are known to effect the oral bioavailability of drug substances. For example, low bioavailability is often the result of low aqueous solubility. Thus, after administration drug substances which are poorly soluble in water tend to be eliminated from the gastrointestinal tract before being absorbed into the bloodstream.

One way of addressing low aqueous solubility is the use of alternative, more powerful solvents such as DMSO. Such solvents, although suitable for pharmacology studies, are rarely suitable for general clinical use. It is well known that the rate of dissolution of a particulate drug can be inversely proportional to the particle size of the drug, i.e. the rate of solubility increases with increasing surface area. Consequently, an alternative strategy to increase the bioavailability of poorly soluble drugs is to prepare them as finely divided compositions. A number of methods for reducing drug particle size are known in the art.

Two such methods of fluid energy milling (micronising) are opposed jet (fluidised bed type) or spiral jet (pancake type). These methods are favoured because of the reduced risk of introducing unfavourable contamination into the drug from mill materials, size reduction being caused by particle-particle collisions. However, the smallest particle size achievable by either of these methods is in the range of 2 -5 microns in diameter. Dry milling methods (such as hammer milling) have also been used to reduce drug particle size and hence increase drug solubility. However, the smallest particle size obtainable is approximately 30 microns in diameter. Although these particle sizes are appropriate for tablet formation and other formulation types, the degree of division is not fine enough to significantly increase the rate of dissolution for poorly soluble drugs.

Another technique for finely dividing preparations is wet milling. Conventional wet milling techniques comprise subjecting a liquid suspension of coarse drug substance to mechanical means, such as a dispersion mill, for reducing the size of the drug substance. One example of a dispersion mill is a media mill, such as a bead mill. Wet bead milling involves preparing a suspension of unmilled coarse drug substance. This dispersion is then drawn through a mill chamber containing a motor driven paddle and a quantity of grinding beads, to produce a finely milled suspension. A screen is used to retain the beads within the mill chamber whilst allowing the passage of product out of each mill chamber. Inline mixers may be used in the process line to break up milled/unmilled agglomerates.

Most wet bead milling is carried out using a re-circulation process through one mill chamber, with one bead size being used to achieve the necessary size reduction. This is an established process for paint, ink and ceramic processing where a fixed amount of energy [in kW/hours] is fed into the product during the wet milling process to meet a target particle size. The mills used for wet milling commonly employ toughened ceramic or stainless steel e.g. tungsten carbide to form the mill chambers and agitating paddles, and commonly used grinding media include the newly developed yttrium stabilised zirconium oxide beads, which have a hardness approaching that of diamonds, or considerably softer grinding media based on polystyrene or other similar polymers.

Contamination of the product by the grinding media and mill chambers is a problem commonly encountered with wet milling. In large scale batches (>10Kg), to achieve a particle size of less than 1 micron, grinding media contamination levels (zirconium and yttrium, plus the elements that form stainless steel e.g. iron, vanadium, etc.) can increase beyond 250ppm. Such levels of contamination are clearly unacceptable in the preparation of pharmaceuticals. One way of avoiding this problem is to use polystyrene based grinding beads. However, this has the disadvantage that process times for large batches (i.e. >20Kg) can be several days. An alternative approach has been to coat milling surfaces of the wet bead mill with polyurethane (Netzsch Feinmahltechnik GmbH). However, mill components coated with polyurethane have been found in practice to have a very short life span, being easily damaged by the grinding media used in the wet milling process.

U.S. Patent no. 5,145,684 and European Patent Application EP-A-0 499 299 disclose a wet milling procedure to produce particles of a crystalline drug substance having a surface modifier adsorbed on the surface in an amount sufficient to maintain an effective average particle size (D₉₅ - D₉₉) of less than about 400 nm. This particulate composition as a stable suspension is said to provide improved bioavailability for poorly water soluble compounds. However, the process itself is very long, often exceeding 24 hours and high contamination levels from grinding media and mill components are experienced. Thus, in EP-A-0 499 299 contamination levels of silicone from glass grinding beads are measured at 10ppm, 36ppm and 71ppm in an aqueous slurry of wet milled danazol (Examples 3, 4, and 5 respectively). This equates to levels of 38ppm, 102ppm and 182ppm in an equivalent dry formulation respectively.

WO 99/30687 (SmithKline Beecham) discloses inter alia compositions comprising benzopyran compounds (such as *trans*-6-acetyl-4S-(4-fluorobenzoylamino)-3,4-dihydro-2,2-dimethyl-2H-1-benzopyran-3R-ol and *cis*-6-acetyl-4S-(3-chloro-4-fluorobenzoylamino)-3,4-dihydro-2,2-dimethyl-2H-1-benzopyran-3S-ol) in particulate form, having a particle size distributions such that the median value of the volume mean diameter is within the range of from 350 to 700nm. One method described in WO 99/30687 as being suitable for preparing these compositions involves wet milling an aqueous dispersion in a bead mill, in which the chambers of the mill are lined with or constructed from an abrasion-resistant polymer material such as nylon. Such a method is stated as having the advantage of reducing contamination from mill materials. The examples of WO 99/30687 describe milled preparations having levels of contamination from yttria-stabilised zirconium powder grinding beads: <200ppm in the case of zirconium and <20ppm in the case of yttrium.

It is therefore an object of the present invention to provide an improved wet milling process suitable for preparing finely divided pharmaceutical compositions, in which contamination of the product is reduced without compromising process speed.

It has surprisingly been found that a wet milling procedure using a mill in which at least some of the milling surfaces are made of nylon (polyamide) comprising one or more internal lubricants not only results in a milled product with dramatically reduced contamination levels from the mill grinding media, but also eliminates contamination from all of the mill chamber component materials as well, without compromising process efficiency.

Accordingly, in first aspect the present invention provides a process for preparing a finely divided preparation of a drug substance comprising wet milling a suspension of the drug substance in a mill having at least one chamber and agitation means, said chamber(s) and/or said agitation means comprising an internally lubricated nylon..

The process of the present invention uses a wet milling step carried out in a mill such as a dispersion mill in order to produce a finely divided particulate suspension of a drug substance. The present invention may be put into practice using a conventional wet milling technique, such as those described in Lachman *et al*., The Theory and Practice of Industrial Pharmacy, Chapter 2, "Milling" p.45 (1986). The suspension of the drug substance for use in the wet milling is typically a liquid suspension of the coarse drug substance in a liquid medium. By "suspension" is meant that the drug substance is essentially insoluble in the liquid medium. Suitably an aqueous medium can be used. The coarse drug substance may be obtained commercially or prepared by techniques known in the art. Using the process of the present invention the average particle size of the coarse drug preparation may be up to 1mm in diameter. This advantageously avoids the need to pre-process the drug substance.

An aqueous medium suitably contains one or more pharmaceutically acceptable water-soluble carriers which are suitable for steric stabilisation and the further processing of the drug substance after milling to a pharmaceutical composition, e.g. by spray drying. Pharmaceutically acceptable excipients most suitable for steric stabilisation and spray-drying are surfactants such as poloxamers, sodium lauryl sulphate and polysorbates etc; stabilisers such as celluloses e.g. hydroxypropylmethyl cellulose; and carriers such as carbohydrates e.g. mannitol.

In the aqueous medium to be subjected to the milling, the drug substance may be present from about 1% to about 40% w/v.

The amount of the primary stabilising agent such as hydroxypropylmethyl cellulose (HPMC), may vary from about 0.1 to about 5% w/v of the composition to be milled. The amount of carrier may vary from 1 to 10% w/v.

Mills suitable for use in the present invention include dispersion mills such as ball mills, attritor mills, vibratory mills and media mills such as sand mills and bead mills. Dispersion mills such as these are well known in the art. A dispersion mill suitable for use in the present invention would comprise at least one mill chamber unit, defining an internal chamber and having within the internal chamber means for agitating the substance to be milled and the grinding media. The dispersion mill may comprise a single mill chamber unit, or alternatively a plurality of mill chamber units. In the latter case the mill chambers could be arranged in sequence such that during milling the liquid suspension of drug substance is passed via fluid connections through one, some or all of the chambers in a sequential manner. In either case the drug substance may be processed through the dispersion mill in a single pass or by re-circulating the drug substance through the mill a desired number of times i.e. a multipass process. A single pass process is preferred. References herein below to "chamber" and "chambers" include a reference to one chamber or more than one chamber selected from the total number of chambers in a mill.

In the case of media mills the agitation may be achieved by paddles, pins, discs etc. moveably mounted within the mill chamber, for example on a rotating shaft driven by an external motor. Grinding means suitable for use in a media mill in the process of the present invention may be a medium such as sand or beads, but for the preparation of a finely milled drug substance beads are recommended.

"Nylon" means a polyamide and includes Nylon 6, Nylon 6,6, Nylon 4,6, Nylon 11 and Nylon 12. High molecular weight nylon is preferred. Suitable high molecular weight nylons for use in the present invention include nylons having a weight average molecular weight of greater than about 30,000Da. Favourably, the high molecular weight nylon has a weight average molecular weight of greater than about 100,000 Da.

By "lubricated nylon" is meant a nylon containing a lubricant such as a plasticising lubricant, which lubricant is distributed through the nylon. Suitable lubricants include low molecular weight hydrocarbon lubricants, such as phthalates e.g. dihexyl phthalate, diisooctyl phthalate, diisononyl phthalate and diisononyl adipate; and higher molecular weight plasticisers such as petroleum wax. Lubricants may be in liquid or solid form e.g. oils or waxes, or a combination thereof.

To achieve the advantages of the present invention it is envisaged that at least the surfaces of the chamber and/or the surfaces of the agitation means which make contact with the drug substance and the grinding media during the milling process are made of lubricated nylon. Thus, the chamber and/or agitation means may be moulded entirely of lubricated nylon, or they may be made of conventional materials with a lubricated nylon insert or coated with a complete or partial layer of lubricated nylon.

In a preferred embodiment of this aspect of the invention the chamber(s) and agitation means of the dispersion mill comprise lubricated nylon. Thus, at least the surfaces of the chambers and the surfaces of the agitation means which make contact with the drug substance and the grinding media during the milling process are made of lubricated nylon.

The lubricated nylon may advantageously comprise one or more liquid or solid lubricants or a combination of liquid and solid lubricants. Particularly good results are achieved when the nylon comprises a combination of liquid and solid lubricants. Advantageously, the nylon may comprise 1, 2, 3, 4, 5 or 6 different lubricants.

Preferably the lubricated nylon (such as a high molecular weight lubricated nylon) will have at least one of the following characteristics and preferably all of them:
- Shore D hardness at 23°C of 70-90, more preferably 80-85
- Compression strength at 23°C of 650-810 kg/cm²; or 80-120 N/mm², more preferably 85-100 N/mm²
- Flexural strength at 23°C of 700-1270 kg/cm²
- Coefficient of friction (sample on steel) of ≤0.5, more preferably ≤0.3, still more preferably ≤0.2, most preferably ≤0.1. (Typically the coefficient of friction will be in the range of 0.08 to 0.4.)
- Tensile strength at 23°C of 710-920 kg/cm²; or ≥35 N/mm², more preferably 40-100 N/mm², most preferably 60-90 N/mm²
- Tensile impact of 650-1100 joule/cm²
- Wear loss of ≤1mg/km under test conditions of 55m(min)⁻¹.MPa, preferably ≤0.7mg/km, more preferably ≤0.4mg/km, even more preferably ≤0.1mg/km.

Particular commercial products which have these characteristics include the high molecular weight nylons Nylube™, Oilon™ and Natural 6™, all available from Nylacast Ltd. *supra*. A particularly preferred lubricated nylon is Nylube™ available from Nylacast, which comprises a solid lubricant and has the following characteristics:
- Shore D hardness at 23°C of 80-84 (ASTM D638)
- Compression strength at 23°C of 650-800 kg/cm² (BS303)
- Flexural strength at 23°C of 700-1200 kg/cm² (BS303)
- Coefficient of friction of 0.08 to 0.10 (nylon on steel)
- Tensile strength at 23°C of 710-890 kg/cm² (ASTM D638)
- Tensile impact of 650-1050 joule/cm² (ASTM D676)
- Wear loss of ≤0.1mg/km under test conditions of 55m(min)⁻¹.MPa

A particularly preferred type of Nylube™ is Nylube CF016™ which under test conditions of 55m(min)⁻¹.MPa typically has a wear loss of 0.02mg/km.

Another particularly preferred lubricated nylon is Oilon™ availabe from Nylacast, which comprises a liquid lubricant and has the following characteristics:
- Shore D hardness at 23°C of 80-85 (ASTM D638)
- Compression strength at 23°C of 670-810 kg/cm² (BS303)
- Flexural strength at 23°C of 770-1270 kg/cm² (BS303)
- Coefficient of friction of 0.13 to 0.14 (nylon on steel)
- Tensile strength at 23°C of 720-900 kg/cm² (ASTM D63.8)
- Tensile impact of 660-1100 joule/cm² (ASTM D676) Wear loss of ≤0.1mg/km under test conditions of 55m(min)⁻¹.MPa

Another preferred lubricated nylon is Nyloil-FG available from Cast Nylons, USA.

The use of Nylacast's Nylube CF016™ is particularly preferred in the process of the present invention because of the almost negligible wear at very high loadings.

Preferably, the dispersion mill used in the process of the present invention is a bead mill. A suitable bead mill is the AP0010 mill from Nylacast Ltd., Leicester, UK. Bead mills manufactured by others such as Dena Systems BK Ltd., Barnsley, UK or Drais, GmbH, Mannheim, Germany could also be used for wet milling drug substances.

In this embodiment the agitation means suitably comprise paddles, pins or discs or any combination of these. A favoured agitation means is one or more rotating paddles. The beads may be made from polystyrene, glass, zirconium oxide stabilised with magnesia, zirconium oxide stabilised with yttrium, zirconium oxide stabilised with cerium, zirconium silicate, zirconia-alumina, stainless steel, titanium or aluminium. Particularly suitable for use in the present invention are beads made of zirconium oxide stabilised with yttrium. Beads suitable for use in this embodiment of the invention such as those listed above are available in a variety of sizes. Generally, spherical beads having mean diameter of up to about 5mm may be employed, but good results are achieved when the beads have a mean diameter of less than 2mm, preferably about 0.1 to about 1.25mm.

In this aspect of the invention, preferably a mill comprising a plurality of mill chambers is used. These chambers should be in fluid connection with each other as described above. For example, a bead mill may comprise 2-10 mill chambers, the precise number of mill chambers being selected to optimise process time and depending on the size of the drug particles both in the coarse suspension of the drug substance and desired in the resulting milled preparation. Variable bead loadings and/or motor speeds are selected to optimise the milling process.

In embodiments of the invention in which the dispersion mill is a bead mill with a plurality of mill chambers, additional advantages are achieved if the average diameter of the grinding beads in a first mill chamber is less than the average diameter of the grinding beads in a second mill chamber, wherein the second mill chamber is upstream of the first mill chamber. For example, the average diameter of the grinding beads in the first mill chamber may be larger than the average diameter of the beads in the following mill chamber. In a particularly preferred embodiment, the average diameter of the beads is reduced in successive mill chambers, i.e. each mill chamber contains on average similar sized or smaller beads than the preceding mill chamber. This enables smaller particle sizes of drug substance to be achieved without an increase in the level of contamination from the grinding media or chamber.

In embodiments of the invention in which the dispersion mill is a bead mill with a plurality of mill chambers the drug substance may be circulated through all of the chambers. Alternatively, by isolating one or more of the mill chambers the number of mill chambers through which the drug substance is circulated may be reduced to one or some of the total number of mill chambers in the bead mill. Regardless of the number of mill chambers through which the drug substance is circulated, the drug substance may be passed through the bead mill just once before being further processed, or a number of times. In other words, the drug substance may be wet milled in a single pass or a multipass process. In multi-pass processes the number and/or order of mill chambers through which the drug substance is circulated may vary from cycle to cycle. Preferably, the drug substance is circulated through all of the chambers in sequence only once. This one-pass process offers the advantages of decreased processing time and minimised contact of the drug substance with the grinding beads and the chamber surfaces, thereby reducing contamination.

The process of the present invention may comprise the further step of drying the drug substance. By "drying" is meant the removal of any water or other liquid vehicle used during the process to keep the drug substance in liquid suspension or solution. This drying step may be any process for drying known in the art, including freeze drying, spray granulation or spray drying. Of these methods spray drying is particularly preferred. All of these techniques are well known in the art. Spray drying/fluid bed granulation of milled compositions is carried out most suitably using a spray dryer such as a Mobile Minor Spray Dryer [Niro, Denmark], or a fluid bed drier, such as those manufactured by Glatt, Germany.

In second aspect the present invention provides a finely divided preparation of a drug substance obtainable by the process according to the first aspect of the invention. In this aspect of the invention the effective average particle size (D₉₅ - D₉₉) of the preparation typically is less than about 3000nm, such as in the range of 400 nm to about 2500 nm. Frequently the effective average particle size of the preparation is in the range of 450 to 1200 nm. The particle size distributions of the suspension formulations may be determined by a number of analytical techniques such as laser diffraction or photon correlation spectroscopy. For example, a Malvern laser diffraction unit, Master Sizer S Model S4700, from Malvern Instruments Ltd., Malvern, England may be employed to characterise finely divided suspensions, or a photon correlation spectroscopy instrument such as the Malvern Zetasizer 5000, also from Malvern Instruments Ltd., Malvern, England may be employed to characterise finely divided suspensions. In addition, any other particle size technique with sufficient sensitivity and resolution for nanoparticulates can be used.

In this aspect of the invention the level of grinding media contamination in the solid (dried) drug preparation, for example a spray dried powder, is typically ≤20 ppm, more typically ≤10ppm, even more typically ≤5ppm. For a wet milled drug preparation present at concentrations of between 1 and 30% w/w in an aqueous slurry with between 0.1 and 10% w/w of stabiliser in the aqueous slurry, these contamination levels typically equate to between 8 and 0.2ppm, more typically between 4 and 0.1ppm and even more typically 2 and 0.05ppm.

An unexpected advantage of the present invention is that drug preparations prepared using the milling process of the present invention do not contain detectable levels of contamination from the mill components (the level of quantification being 0.1ppm).The total level of contamination from the milling process has been investigated, and surprisingly contributions from the polymeric components of the mill are substantially less than 0.1ppm, hence the total process contamination is typically ≤20 ppm, preferably ≤10ppm, more preferably ≤5ppm.

In this aspect of the invention the drug substance may be, for example, nabumetone or *trans*-6-acetyl-4S-(4-fluorobenzoylamino)-3,4-dihydro-2,2-dimethyl-2H-1-benzopyran-3R-ol.

In third aspect the present invention provides a pharmaceutical composition comprising a finely divided preparation of a drug substance prepared according to the process of the invention. Compositions are prepared by admixture and, thus, they are suitably adapted for oral or parenteral administration. The compositions may be in the form of tablets, capsules, reconstitutable powders or suppositories. Orally administerable compositions are preferred.

Tablets and capsules for oral administration are usually presented in a unit dose, and contain conventional excipients such as binding agents, fillers and diluents (tableting or compression aids), lubricants, disintegrants, colorants, flavourings, and wetting agents. The tablets may be coated according to techniques well known in the art.

The solid oral compositions may be prepared by conventional methods of blending, filling, tableting, or the like. Repeated blending operations may be used to distribute the active agent throughout those compositions employing large quantities of fillers. Such operations are, of course, well known in the art.

Oral formulations also include conventional controlled release formulations, such as tablets or pellets, beads or granules, having a sustained release or an enteric coating, or otherwise modified to control the release of the active compound, for example by the inclusion of gel forming polymers or matrix forming waxes.

Advantageously, a wetting agent is included in the composition to facilitate uniform distribution of the compound of the invention.

The compositions of the invention are preferably adapted for oral administration. The compositions are preferably presented as a unit dose. Such a composition is taken preferably from 1 to 2 times daily. The preferred unit dosage forms include tablets or capsules. The compositions of this invention may be formulated by conventional methods of admixture such as blending, filling and compressing. Suitable pharmaceutically acceptable carriers for use in this invention include diluents, fillers, binders and disintegrants.

For a better understanding of the present invention and to illustrate how the same may be put into effect, reference will now be made, by way of example, to the accompanying drawings, in which:
Figure 1 is a dispersion mill which may be used in accordance with a preferred embodiment of the present invention.
Figure 2 is an alternative mill arrangement.

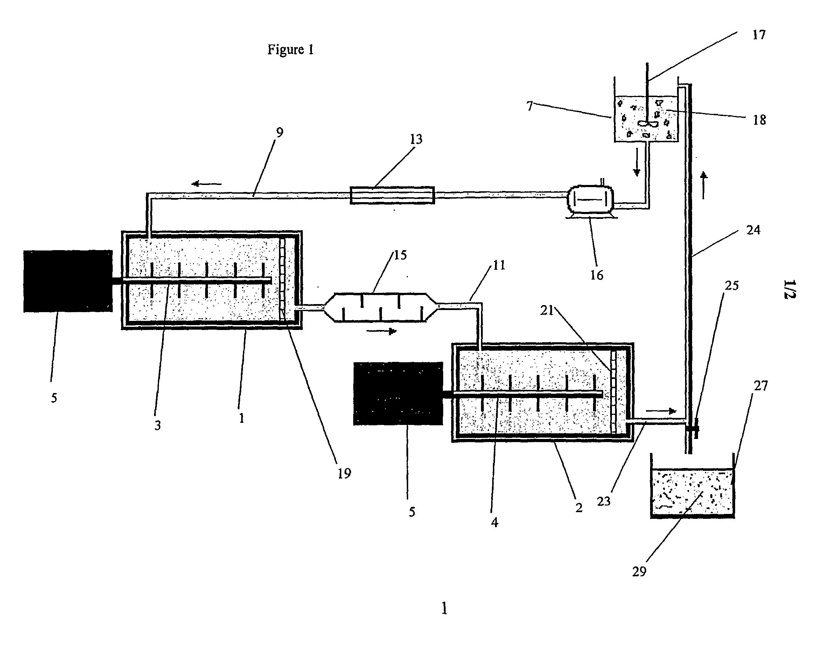

With reference to Figure 1, a mill in accordance with the present invention comprises two mill chambers (1, 2) each having a paddle (3) driven by a motor (5). The chambers (1, 2) and paddles (3, 4) are moulded from Nylube CF016. The first chamber is in fluid connection with a reservoir (7) and the second chamber (2) via pipes (9, 11). Each pipe (9, 11) is fitted with an-in line mixer (13, 15). The pipe connecting the reservoir and the first chamber (9) is also fitted with suitable pump such as an air pump (16) which is powerful enough to pump liquid medium around the whole mill. The reservoir contains a mixing device (17), which in use maintains a liquid suspension of the coarse drug substance (18). Each mill chamber (1, 2) contains a quantity of yttrium stabilised zirconium oxide beads (not shown) which are retained by screens (19, 21). An exit pipe (23) links the second mill chamber (2) to a recirculation pipe (24) connected to the reservoir (7). The recirculation pipe (24) contains a tap (25). A collection reservoir (27) is provided to collect the nano-milled drug suspension (29).

In use, the reservoir (7) is charged with coarse drug substance in a liquid medium (18) and maintained in suspension by the mixing device (17). The suspension of the coarse drug substance is pumped by the air pump (16) along the pipe (9) through the first in-line mixer (13), which removes agglomerates from the suspension. The superfine dispersion then enters the first mill chamber (1). In the first mill chamber the combined action of the paddle (3) as it is driven by the motor (5) and the beads (not shown) grinds the coarse drug suspension for a pre-set duration which is controlled by the operation of the pump (16). This partly milled dispersion is then pumped through a further in-line mixer (15) and the second mill chamber (2) before exiting the second mill chamber through exit pipe (23). This nano-milled suspension of drug substance (29) may then be either recirculated back into the first reservoir (7) via the recirculation pipe (24) or, if the tap (25) is opened, drained into the collection reservoir (27).

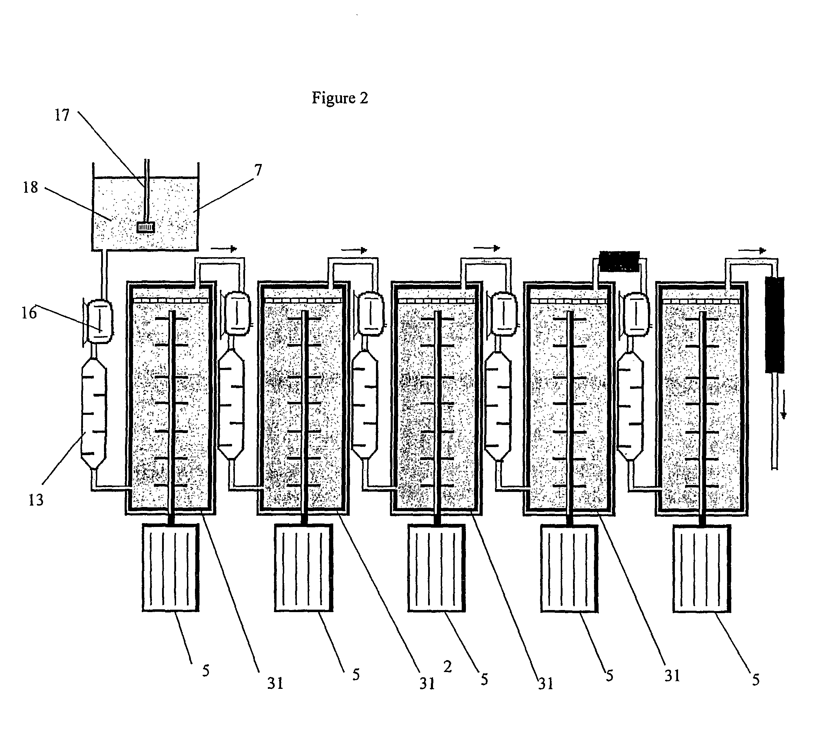

In an alternative mill arrangement, an equal number of mill chambers (31) and air pumps (16) are arranged in series (see Figure 2).

The following examples are illustrative of the instant invention.

### Examples

### Example 1

A 200Kg batch of an aqueous suspension comprising 20% w/w of 6-Acetyl-3,4-dihydro-2,2-dimethyl-trans(+)-4-(4-fluorobenzoylamino)-2H-benzo[b]pyran-3-ol (for preparation see Example 20 of WO 92/22293), 1.5% w/w hydroxypropyl methyl cellulose, 0.2% w/w sodium lauryl sulphate and 5.0% w/w mannitol was passed through a Dena DS-1P5 bead mill. Five 8L mill chambers fabricated from Nylacast Nylube were used in a single pass configuration, with each chamber containing 85% by volume of yttrium stabilised zirconium oxide beads (from Tosoh, Japan). The following bead sizes were employed: Chambers one through to five contained 1.0mm, 0.8mm, 0.65mm, and 2 chambers with 0.4mm respectively. The batch was processed at 2.9L per minute, with a product dwell time within the mill of 5 minutes and a batch processing time of 70 minutes. Chamber pressures during processing varied between 2 and 3 bar [28 to 42 psi]. The yield exceeded 85%. The finely milled suspension was subsequently spray dried.

Grinding media contamination levels in the spray dried powder were < 3ppm Zirconium (Zr) and < 1ppm Yttrium (Y).

The unprocessed particle size of the drug was approximately 1mm, and the product had a median particle size of 0.5 microns as measured by refractive index corrected laser diffraction.

### Example 2

A 200Kg batch of an aqueous suspension containing 30% w/w of 4-(6'-methoxy-2'-naphthyl)-butan-2-one (nabumetone, for preparation see US Patent No. 4,420,639), w/w sodium lauryl sulphate, 3% w/w hydroxypropyl methyl cellulose and 4% w/w mannitol was passed through a Dena DS-1P5 bead mill. Five 8L mill chambers fabricated from Nylacast Nylube were used in a single pass configuration, with each chamber containing 70% by volume of yttrium stabilised zirconium oxide beads (from Tosoh, Japan). The following bead sizes were employed: Chambers one through to five contained 1.0mm, 0.8m, 0.65mm, and 2 chambers with 0.4mm respectively. The batch was processed at 1.5L per minute, with a product dwell time within the mill of 10 minutes and a batch processing time of 2 ¼ hours. Chamber pressures during processing varied between 2 and 3 bar [28 to 42 psi]. The yield exceeded 85%. The finely milled suspension was subsequently spray dried.

Grinding media contamination levels in the spray dried powder were < 3ppm Zirconium (Zr) and < 1ppm Yttrium (Y).

The unprocessed particle size of the drug was approximately 1mm, and the product had a median particle size of 0.9 microns as measured by laser diffraction.

An investigation into potential product contamination from polymer based mill components by the Rubber And Plastic Research Association (Shawbury, UK) was made using rigorous extraction procedures and analysis by Gas Chromotography, High Pressure Liquid Chromotography and Mass Spectrometry. The component parts included the nylon mill chamber and paddles; PTFE, Viton and EPDM O-rings, and the PEEK filled PTFE gap separator. Although several extractable species could be identified, analysis of the spray dried powder found that there was no product carry over of any mill component species. The limit of quantification for each extractable species was 40ppb and the limit of detection was 20ppb. The total amount of extracted species in the spray dried product are less than 0.1 ppm

## Claims

1. Process for preparing a finely divided preparation of a drug substance comprising wet milling a suspension of the drug substance in a mill having at least one chamber and agitation means, said chamber(s) and/or said agitation means comprising internally lubricated nylon.

2. The process as claimed in claim 1 wherein said chamber and said agitation means comprise internally lubricated nylon.

3. The process as claimed in claim 1 or claim 2 wherein the lubricated nylon comprises one or more solid lubricants.

4. The process as claimed in any preceding claim wherein the lubricated nylon comprises one or more liquid lubricants.

5. The process as claimed in any preceding claim wherein the lubricated nylon comprises more than one lubricant.

6. The process according to any preceding claim wherein the lubricated nylon has a coefficient of friction of ≤0.35.

7. The process as claimed in any preceding claim wherein the lubricated nylon is Nylube™, Oilon™, or Nyloil-FG™.

8. The process according to any one of the preceding claims which further comprises the step of drying the drug substance.

9. A finely divided preparation of a drug substance obtainable by the process of any one of claims 1 to 8 wherein the level of grinding media contamination in the solid dried drug preparation is ≤20 ppm.

10. The finely divided preparation of claim 9 wherein the level of grinding media contamination is ≤10ppm.

11. The finely divided preparation of claim 9 wherein the level of grinding media contamination is ≤5ppm.

12. The finely divided preparation of claim 9 wherein the total level of process contamination is ≤20 ppm.

13. The finely divided preparation of claim 9 wherein the total level of process contamination is ≤10ppm.

14. The finely divided preparation of claim 9 wherein the total level of process contamination is ≤5ppm.

15. A pharmaceutical composition comprising a finely divided preparation of a drug substance as claimed in any one of claims 9 to 14.

16. A finely divided preparation as claimed in any one of claims 9 to 14 or a composition as claimed in claims 15 wherein the drug substance is nabumetone or *trans*-6-acetyl-4S-(4-fluorobenzoylamino)-3,4-dihydro-2,2-dimethyl-2H-1-benzopyran-3R-ol.

## Patentansprüche

1. Verfahren zur Herstellung einer fein vermahlenen Zubereitung eines Arzneistoffes umfassend Nassvermahlen einer Suspension des Arzneistoffes in einer Mühle, welche mindestens eine Kammer und eine Rührvorrichtung aufweist, wobei die Kammer(n) und/oder die Rührvorrichtung Nylon mit eingearbeitetem Gleitmittel umfasst (umfassen).

2. Verfahren gemäß Anspruch 1, wobei die Kammer und die Rührvorrichtung Nylon mit eingearbeitetem Gleitmittel umfassen.

3. Verfahren gemäß Anspruch 1 oder Anspruch 2, wobei das geschmierte Nylon ein oder mehrere feste Gleitmittel umfasst.

4. Verfahren gemäß einem der vorstehenden Ansprüche, wobei das geschmierte Nylon ein oder mehrere flüssige Gleitmittel umfasst.

5. Verfahren gemäß einem der vorstehenden Ansprüche, wobei das geschmierte Nylon mehr als ein Gleitmittel umfasst.

6. Verfahren gemäß einem der vorstehenden Ansprüche, wobei das geschmierte Nylon einen Reibungskoeffizienten von ≤ 0,35 aufweist.

7. Verfahren gemäß einem der vorstehenden Ansprüche, wobei das geschmierte Nylon Nylube™, Oilon™ oder Nyloil-FG™ ist.

8. Verfahren gemäß einem der vorstehenden Ansprüche, welches weiterhin den Schritt Trocknen des Arzneistoffes umfasst.

9. Fein vermahlene Zubereitung eines Arzneistoffes, welche durch das Verfahren nach einem der Ansprüche 1 bis 8 erhältlich ist, wobei der Verunreinigungsgrad der Mahlkörper in der festen, trockenen Arzneimittelzubereitung ≤ 20 ppm beträgt.

10. Fein vermahlene Zubereitung gemäß Anspruch 9, wobei der Verunreinigungsgrad der Mahlkörper ≤ 10 ppm ist.

11. Fein vermahlene Zubereitung gemäß Anspruch 9, wobei der Verunreinigungsgrad der Mahlkörper ≤ 5 ppm ist.

12. Fein vermahlene Zubereitung gemäß Anspruch 9, wobei der gesamte Verunreinigungsgrad in dem Verfahren ≤ 20 ppm ist.

13. Fein vermahlene Zubereitung gemäß Anspruch 9, wobei der gesamte Verunreinigungsgrad in dem Verfahren ≤ 10 ppm ist.

14. Fein vermahlene Zubereitung gemäß Anspruch 9, wobei der gesamte Verunreinigungsgrad in dem Verfahren ≤ 5 ppm ist.

15. Arzneimittel, welches eine fein vermahlene Zubereitung eines Arzneistoffes nach einem der Ansprüche 9 bis 14 umfasst.

16. Fein vermahlene Zubereitung gemäß einem der Ansprüche 9 bis 14 oder Zusammensetzung nach Anspruch 15, wobei der Arzneistoff Nabumeton oder *trans*-6-Acetyl-4S-(4-fluorbenzoylamino)-3,4-dihydro-2,2-dimethyl-2H-1-benzopyran-3R-ol ist.

## Revendications

1. Procédé pour la production d'une préparation finement divisée d'une substance médicamenteuse, comprenant le broyage à l'état humide d'une suspension de la substance médicamenteuse dans un broyeur comprenant au moins une chambre et un moyen d'agitation, ladite ou lesdites chambres et/ou ledit moyen d'agitation comprenant un Nylon à lubrification interne.

2. Procédé suivant la revendication 1, dans lequel ladite chambre et ledit moyen d'agitation comprennent un Nylon à lubrification interne.

3. Procédé suivant la revendication 1 ou la revendication 2, dans lequel le Nylon lubrifié comprend un ou plusieurs lubrifiants solides.

4. Procédé suivant l'une quelconque des revendications précédentes, dans lequel le Nylon lubrifié comprend un ou plusieurs lubrifiants liquides.

5. Procédé suivant l'une quelconque des revendications précédentes, dans lequel le Nylon lubrifié comprend plus d'un lubrifiant.

6. Procédé suivant l'une quelconque des revendications précédentes, dans lequel le Nylon lubrifié a un coefficient de frottement ≤ 0,35.

7. Procédé suivant l'une quelconque des revendications précédentes dans lequel le Nylon lubrifié est le Nylube™, le Oilon™ ou le Nyloil-FG™.

8. Procédé suivant l'une quelconque des revendications précédentes, qui comprend en outre l'étape de séchage de la substance médicamenteuse.

9. Préparation finement divisée d'une substance médicamenteuse pouvant être obtenue par le procédé suivant l'une quelconque des revendications 1 à 8, dans lequel le taux de contamination par le milieu de broyage dans la préparation de substances médicamenteuses séchées solides est ≤ 20 ppm.

10. Préparation finement divisée suivant la revendication 9, dans laquelle le taux de contamination par le milieu de broyage est ≤ 10 ppm.

11. Préparation finement divisée suivant la revendication 9, dans laquelle le taux de contamination par le milieu de broyage est ≤ 5 ppm.

12. Préparation finement divisée suivant la revendication 9, dans laquelle le taux total de contamination du procédé est ≤ 20 ppm.

13. Préparation finement divisée suivant la revendication 9, dans laquelle le taux total de contamination du procédé est ≤ 10 ppm.

14. Préparation finement divisée suivant la revendication 9, dans laquelle le taux total de contamination du procédé est ≤ 5 ppm.

15. Composition pharmaceutique comprenant une préparation finement divisée d'une substance médicamenteuse suivant l'une quelconque des revendications 9 à 14.

16. Préparation finement divisée suivant l'une quelconque des revendications 9 à 14 ou composition suivant la revendication 15, dans laquelle la substance médicamenteuse est la nabumétone ou le *trans*-6-acétyl-4S-(4(fluorobenzoylamino)-3,4-dihydro-2,2-diméthyl-2G-1-benzopyranne-3R-ol.
